# EUROPEAN PATENT SPECIFICATION

(11) **EP 2 064 983 B1**
(45) Date of publication and mention of the grant of the patent: **17.12.2014**
(21) Application number: 07828294.4
(22) Date of filing: 21.09.2007
(51) Int. Cl.: A61B 1/04, A61B 5/00, A61B 1/273

(54) **CAPSULE TYPE ENDOSCOPE, AND GASTER OBSERVATION METHOD**
ENDOSKOP VOM KAPSELTYP UND MAGEN-BEOBACHTUNGSVERFAHREN
ENDOSCOPE DE TYPE CAPSULE, ET PROCÉDÉ D'OBSERVATION DE GASTER

(30) Priority: 22.09.2006 JP 2006257270
(43) Date of publication of application: 03.06.2009
(73) Proprietor: Olympus Medical Systems Corp., Tokyo 151-0072 (JP)
(72) Inventor: FUJITA, Manabu, Shibuya-ku, Tokyo 1510072 (JP)
(74) Representative: von Hellfeld, Axel
(86) International application number: PCT/JP2007/068373
(87) International publication number: WO 2008/035760

(56) References cited:
- WO-A-02/09531
- WO-A1-2006/028281
- WO-A1-2007/077922
- WO-A2-2005/062717
- JP-A- 2004 529 718
- JP-A- 2007 190 361
- US-A- 4 507 115
- US-A1- 2005 284 233

## Description

### TECHNICAL FIELD

The present invention relates to a capsule endoscope which allows an observation of an inside of a subject body in a state of floating on a liquid fed into the subject body and an intra-stomach observing method.

### BACKGROUND ART

Recently, a capsule endoscope including an imaging function and a wireless communication function has appeared in the field of endoscopes. The capsule endoscope has a configuration of travelling inside of organs such as the esophagus, the stomach, and the small intestine (inside of a body cavity) according to their peristalsis and of sequentially capturing images by using the imaging function during an observation period which starts when the capsule endoscope is swallowed for the purpose of an observation (examination) from a mouth of a test subject as a subject body (human body) and ends when it is naturally excreted from a living body of the test subject.

Here, a technology of making a capsule endoscope, whose specific gravity is set to less than one and which is swallowed with a liquid (drinking water), float on the liquid inside the stomach to which the liquid is fed, and enabling an observation of a wall of the stomach is disclosed in Patent Document 1.

Patent Document 1: International Publication Pamphlet No. 02/95351 (PCT National Publication No. 2004-529718)
Patent Document 2: Japanese Patent Application Laid-Open No. H10-213384
Patent Document 3: Japanese Patent Application Laid-Open No. 2004-305635

WO 2002/095351 concerns an in vivo sensing device having a specific gravity of about 1 or a volume to weight ratio that enables it to essentially float. In one embodiment the in vivo sensing device consists of an image sensor system and a buoyant body disposed inside the capsule device and attached to the sensor system such that the sensor system essentially floats on a body lumen liquid due to the buoyant body disposed inside the capsule. In another embodiment, the buoyant body is a balloon-type inflatable buoy. In still another embodiment, the inflatable buoy may contain gas releasing granules such as crystalline sodium bicarbonate which release gas upon contacting liquid so as to inflate the buoy. The granules and liquid are kept separate, and the buoy may be inflated dependent on in vivo conditions such as pH, whereby a gelatin capsule dissolves in liquid present in the stomach, thereby releasing the inflatable buoy.

### DISCLOSURE OF INVENTION

### PROBLEM TO BE SOLVED BY THE INVENTION

The capsule endoscope houses contents including an imaging unit, an illumination unit, a wireless communication unit, a battery, and the like in a capsule casing, and a specific gravity of the contents is usually not less than one in most cases. Therefore, it is necessary to form the capsule casing in a size larger than the required size to make the specific gravity of the capsule endoscope less than one, so that the capsule endoscope has a problem of having a difficulty in swallowing from an oral cavity of the test subject as shown in Patent Document 1.

The present invention has been achieved in view of the foregoing and an object of the present invention is to provide: a capsule endoscope which allows, with a low specific gravity in total in a subject body, an observation in a state of floating on a liquid without impairing a property of an insertion into the subject body even when a specific gravity of contents of the capsule endoscope is high; and an intra-stomach observing method.

### MEANS FOR SOLVING PROBLEM

A capsule endoscope according to claim 1 may include: a capsule casing which contains therein contents including an imaging unit and is inserted into an inside of a subject body; a hollow volume changing unit which is connected to the capsule casing, and changes a connection position with the capsule casing to change a volume of the capsule casing; and an actuator which is connected to the capsule casing and changes the connection position.

In the capsule endoscope, the actuator may operate based on a change in a temperature of an outside of the capsule casing.

In the capsule endoscope, the actuator may be a shape memory alloy spring.

In the capsule endoscope, the connection position may be a storage position where the volume of the capsule casing is minimum and an exposure position where the volume of the capsule casing is not minimum, a specific gravity of the capsule casing with respect to a water may be not less than one at the storage position, and a specific gravity of the capsule casing with respect to the water may be less than one at the exposure position.

In the capsule endoscope, the volume changing unit may have a rigid body of a cylindrical shape and may be connected to the capsule casing to be freely movable inward and outward with respect to the capsule casing.

In the capsule endoscope, the volume changing unit may be formed of a soft member of an accordion shape and connected to be freely stretchable inward and outward with respect to the capsule casing.

In the capsule endoscope, the capsule casing may be constituted by divided two casings, and the volume changing unit may be connected between the two casings.

In the capsule endoscope, each of the two casings may have a bottomed shape.

In the capsule endoscope, the actuator may be formed by a shape memory alloy spring which causes the volume changing unit to be displaced from the storage position to the exposure position at a temperature not more than a predetermined temperature.

The capsule endoscope may further include a sealing member which is formed of a material that dissolves when inserted into the subject body and retains the volume changing unit at the storage position against a memorized shape, which causes the displacement to the exposure position, of the shape memory alloy spring.

In the capsule endoscope, a ventilation hole may be formed in the volume changing unit.

In the capsule endoscope, a center of gravity of the capsule endoscope may be set at a position where the ventilation hole is oriented upward with respect to a gravity direction.

In the capsule endoscope, a sheet member which allows only a gas to pass through may be attached to the ventilation hole.

An intra-stomach observing method includes the steps of making a capsule endoscope before an examination at a predetermined temperature; making a subject swallow the capsule endoscope; making the subject take in a water whose temperature is lower than the predetermined temperature; increasing a volume of the capsule endoscope; and observing an inside of a stomach by the capsule endoscope.

An intra-stomach observing method includes the steps of storing a capsule endoscope under an environment of a predetermined temperature; making a subject swallow the capsule endoscope; making the subject take in a water whose temperature is lower than the predetermined temperature; increasing a volume of the capsule endoscope; and observing an inside of a stomach by the capsule endoscope.

In the intra-stomach observing method, the predetermined temperature may be about 36°C to 40°C and the temperature lower than the predetermined temperature may be about 25°C.

An intra-stomach observing method includes the steps of making a subject swallow a capsule endoscope; making the subject take in a water of a predetermined temperature and filling an inside of a stomach with a liquid; increasing a volume of the capsule endoscope; and observing the inside of the stomach by the capsule endoscope.

### EFFECT OF THE INVENTION

A capsule endoscope has advantageous effects that since an actuator changes a connecting position of a volume changing unit with a capsule casing based on a body temperature of a subject body when the capsule endoscope is inserted into the subject body to change a volume of the casing, a volume condition which does not impair a property of an insertion into the subject body can be realized, when a liquid is arbitrarily fed into the subject body after the capsule endoscope is inserted into the subject body, the actuator changes the connecting position of the volume changing unit with the capsule casing based on a temperature of the liquid to change and increase the casing volume, and thereby a total specific gravity can be reduced and an observation can be performed with the capsule endoscope floating on the liquid even when a specific gravity of contents is high.

### BRIEF DESCRIPTION OF DRAWINGS

FIG. 1 is a view showing an observation of an inside of a subject body by using a capsule endoscope according to a first embodiment.
FIG. 2 is a schematic side view of examples of constitutions respectively before and after a volume increase of the capsule endoscope according to the first embodiment.
FIG. 3 is a schematic side view of examples of constitutions respectively before and after a volume increase of a capsule endoscope according to a second embodiment.

### EXPLANATIONS OF LETTERS OR NUMERALS

- 1: Capsule endoscope
- 2: Subject body
- 3: Water
- 11: Capsule casing
- 11A, 11B: Casing
- 12: Contents
- 13: Volume changing unit
- 13a: Ventilation hole
- 14: Shape memory alloy spring
- 15: Sealing member
- 16: Volume changing unit
- 16a: Ventilation hole

### BEST MODE(S) FOR CARRYING OUT THE INVENTION

A capsule endoscope according to best modes(s) for carrying out the invention will be explained below with reference to the accompanying drawings.

### First Embodiment

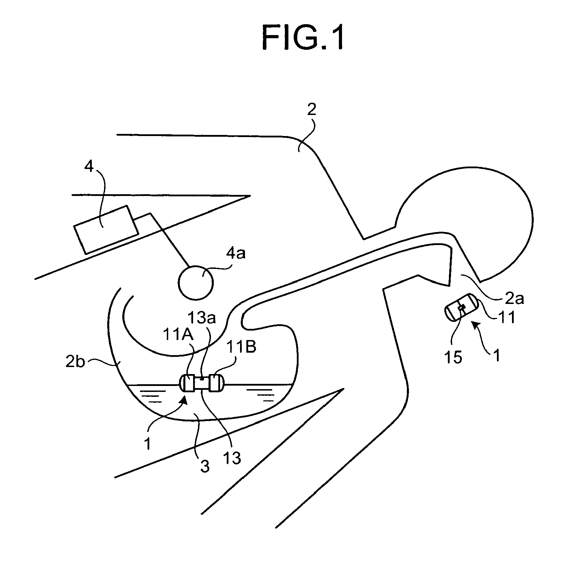

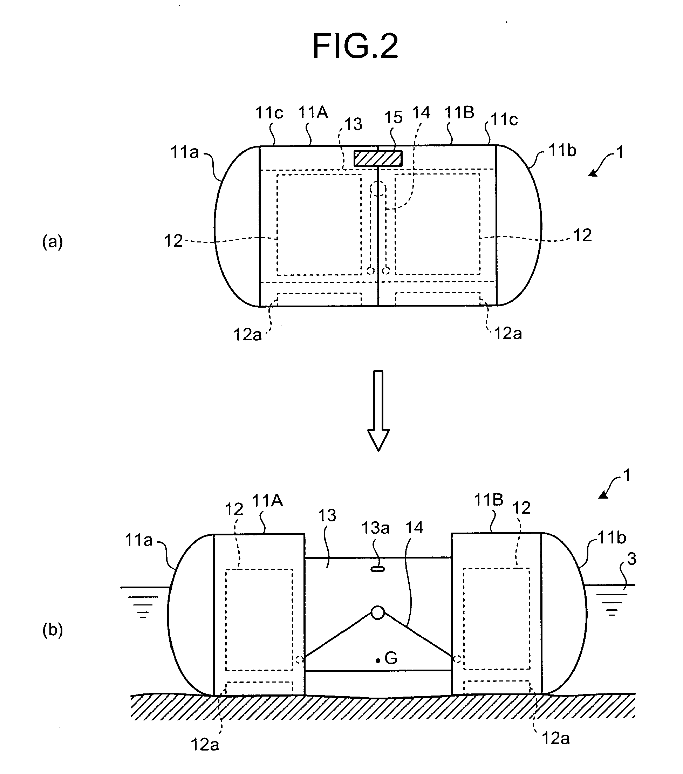

FIG. 1 is a view showing an observation of an inside of a subject body by using a capsule endoscope according to a first embodiment and FIG. 2 is a schematic side view of examples of constitutions respectively before and after a volume increase of the capsule endoscope according to the first embodiment.

As shown in FIG. 1, a capsule endoscope 1 according to the first embodiment is inserted into a subject body 2 by being swallowed from the oral cavity 2a of the subject body 2, and images and observes a stomach wall as a target site by floating on a surface of water 3 whose specific gravity is one in a stomach 2b to which a liquid, for example, the water 3 is fed. A numeral 4 denotes a receiver which receives image data wirelessly transmitted from the capsule endoscope 1 having an imaging function and a wireless communication function. The receiver 4 includes a receiving antenna 4a such as a loop antenna which is attached on an outside surface of the subject body 2, and receives the image data and the like wirelessly transmitted from the capsule endoscope 1 via the receiving antenna 4a.

Here, the capsule endoscope 1 according to the first embodiment is explained by taking, as one example, an application to a compound-eye capsule endoscope which can capture images at both sides in a longitudinal axis direction of the capsule, and includes a capsule casing 11 whose size is small enough to be swallowed from the oral cavity 2a of the subject body 2 and contents 12 including an imaging unit, an illumination unit, a wireless communication unit, a substrate member, a battery, and the like which are embedded in the capsule casing 11.

The capsule casing 11 includes semispherical, transparent or translucent head covers 11a and 11b and a body cover 11c which has a cylindrical shape and is formed of a colored material which does not allow visible light to pass through. The capsule casing 11 according to the embodiment, since the body cover 11c is divided into two pieces in the axial direction, is formed by separated two casings, that is, a first casing 11A and a second casing 11B. Both of the divided surfaces of the divided body cover 11c are configured to be bottomed and to seal insides of the casings 11A and 11B in a liquid-tight manner, respectively.

The illumination unit in the contents 12 is constituted by a light emitting element such as an LED which emits illumination light for illuminating a site to be imaged in the subject body 2 via the head covers 11a and 11b. The imaging unit in the contents 12 includes an imaging element such as a CCD and a CMOS sensor which receives reflection light by the illumination light via the head covers 11a and 11b and captures images of the site to be imaged, an image forming lens, and the like. The contents 12 are separated and embedded into the two casings 11A and 11B respectively to fit a symmetrical structure of the compound-eye capsule endoscope, and each casing includes contents whose specific gravity in total is not less than one and higher than that of the water 3.

In addition to the configuration described above, the capsule endoscope 1 according to the embodiment includes a volume changing unit 13 and a shape memory alloy spring 14 as an actuator which activates the volume changing unit 13. The volume changing unit 13 is schematically a tank having a hollow rigid body of a cylindrical shape, arranged between the casings 11A and 11B with respect to the volume changing unit 13, and connected to be freely movable inward and outward with respect to the casings 11A and 11B along the longitudinal axis direction with an air-tight state with respect to the bottomed parts of the casings 11A and 11B maintained. By this, the volume changing unit 13 can be displaced to a storage position where the volume changing unit 13 is stored half and half by the casings 11A and 11B as shown at (a) in FIG. 2 and to an exposure position where the volume changing unit 13 is exposed outward from the casings 11A and 11B as shown at (b) in FIG. 2 to increase a total casing volume of the capsule endoscope 1 and make the specific gravity less than one. To perform the displacement between the storage position and the exposure position, the volume changing unit 13 is provided, at a center part on a side surface, with one ventilation hole 13a through which an outer air is taken in and an inner air is discharged. Here, a heavy content in the contents 12, for example, a battery 12a is arranged on an inner wall opposite to the ventilation hole 13a in the casings 11A and 11B, so that a center of gravity G of the capsule endoscope 1 is set at a position where the ventilation hole 13a is oriented to an upper direction when the capsule endoscope 1 lies on its side.

The shape memory alloy spring 14, both ends thereof being connected to freely turn with respect to the bottomed parts of the casings 11A and 11B, causes the volume changing unit 13 to be displaced to the storage position by presenting a memorized shape of a folded shape as shown at (a) in FIG. 2 at a temperature around 36°C to 40°C corresponding to a body temperature of the subject body 2 and causes the volume changing unit 13 to be displaced to the exposure position by presenting a memorized shape of an expanded open shape as shown at (b) in FIG. 2 at a temperature around 25°C corresponding to a temperature of the water 3 fed into the subject body 2. The shape memory alloy spring 14 is arranged to the side of the center of gravity G position away from the side of the ventilation hole 13a position.

Besides, the capsule endoscope 1 according to the embodiment includes, on a front surface, a sealing member 15 which seals the casings 11A and 11B to be a connected state so that the volume changing unit 13 is maintained at the storage position against the memorized shape of the shape memory alloy spring 14 that causes the displacement to the exposure position under a condition of a normal temperature before the capsule endoscope 1 is swallowed by the subject body 2. The sealing member 15 is formed of an edible material such as a wafer which dissolves by stomach juices and the like by being inserted into the subject body 2.

In this configuration, since the casings 11A and 11B are sealed by the sealing member 15, the volume changing unit 13 is maintained at the storage position against the memorized shape of the shape memory alloy spring 14 though the capsule endoscope 1 before starting an examination is under the condition of the normal temperature and the shape memory alloy spring 14 will try to present the memorized shape of the expanded open shape when the normal temperature is, for example, at about 25°C. By this, the capsule endoscope 1 can be maintained in a size of the state shown at (a) in FIG. 2 in which the casings 11A and 11B are coupled as one unit without causing the volume changing unit 13 to be exposed. The size is a normal capsule size and does not impair the swallowing performance from the oral cavity 2a.

At a time of starting the examination, the capsule endoscope 1 in the state as shown at (a) in FIG. 2 is swallowed from the oral cavity 2a and inserted into the stomach 2b. Upon the insertion into the subject body 2, the sealing member 15 dissolves by the stomach juices and the like and the sealed state is released. On this occasion, the capsule endoscope 1 is already inserted into the subject body 2 and subjected to a body temperature environment, the shape memory alloy spring 14 presents the memorized shape of the folded shape and therefore the volume changing unit 13 is maintained at the storage position even when the sealed state is released. By this, the capsule endoscope 1 can be maintained in the size of the state shown at (a) in FIG. 2 in which the casings 11A and 11B are coupled as one unit without causing the volume changing unit 13 to be exposed. In this state, the specific gravity of the capsule endoscope 1 is not less than one.

Thereafter, when the time is right, the water 3 at 25°C is taken little by little to be fed into the stomach 2b. On this occasion, though the specific gravity of the capsule endoscope 1 is not less than one and the capsule endoscope 1 does not float on a surface of the water 3 fed into the stomach 2b, the capsule endoscope 1 becomes a state of lying on its side in which the ventilation hole 13a is oriented upward according to the position of the center of gravity G. Then, the shape memory alloy spring 14 of the lying capsule endoscope 1 comes to be present in the fed water 3 (the water 3 is assumed to be an amount not causing the capsule endoscope 1 to go under the water as shown at (b) in FIG. 2 at this point) and performs the displacement to present the memorized shape of the expanded open shape from the folded shape according to the temperature (25°C) of the water 3. In response to the displacement to the expanded open shape of the shape memory alloy spring 14, the casings 11A and 11B are displaced to a direction of becoming away from each other, and the volume changing unit 13 connected between the casings 11A and 11B makes the displacement to the exposure position as shown at (b) in FIG. 2 while absorbing an air through a cylinder effect from the ventilation hole 13a present at a position oriented to an upper direction and in the air in the stomach 2b. Due to the displacement of the volume changing unit 13 to the exposure position, the casing volume of the capsule endoscope 1 increases to be a state where the specific gravity is less than one.

When an appropriate amount of the water 3 is further fed into the stomach 2b under this condition, the capsule endoscope 1 whose specific gravity is lowered to be less than one can image and observe the wall of the stomach while floating on the surface of the fed water 3 as shown in FIG. 1.

After the examination is completed, the water 3 is discharged from the inside of the stomach 2b to a side of the small intestine. By this, the capsule endoscope 1 remaining in the stomach 2b is subjected to the environment of the body temperature of the subject body 2 and the shape memory alloy spring 14 makes the displacement from the expanded open shape to a direction to be folded to present the memorized shape of the bent shape. In response to this folding displacement of the shape memory alloy spring 14, the separated casings 11A and 11B are also displaced to a direction to be in contact with each other and the volume changing unit 13 connected between the casings 11A and 11B also makes the displacement to the storage position as shown at (a) in FIG. 2 while discharging the inner air from the ventilation hole 13a. By the displacement of the volume changing unit 13 to the storage position, the size of the capsule endoscope 1 returns to a normal capsule size. Then, the capsule endoscope 1 moves to the side of the small intestine according to the subsequent peristalsis as usual and is finally excreted to an outside of the subject body 2.

As described, since the shape memory alloy spring 14 presents the memorized shape of causing the volume changing unit 13 to be displaced to the storage position where the volume changing unit 13 is stored in the capsule endoscope 1 based on the body temperature of the subject body 2 when the capsule endoscope 1 according to the embodiment is inserted into the subject body 2, a volume condition which does not impair the insertion property into the subject body 2 can be realized. Besides, since the shape memory alloy spring 14 presents the memorized shape of causing the volume changing unit 13 to be displaced to the exposure position where the volume changing unit 13 is exposed to the outside of the casing of the capsule endoscope 1 and the casing volume is increased based on the temperature of the water 3 fed appropriately into the subject body 2 after the capsule endoscope 1 is inserted into the subject body 2, the volume changing unit 13 makes the displacement to the exposure position to increase the casing volume while absorbing the outer air into the inside through the ventilation hole 13a in the subject body 2, so that an observation in a state where the capsule endoscope 1 with a low specific gravity in total floats on the water 3 can be performed even when the specific gravity of the contents 12 is high.

In addition, a common linear actuator may be provided instead of the shape memory alloy spring 14. A temperature sensor may further be equipped for controlling an operation of the linear actuator based on a temperature. By this, the volume changing unit 13 can be controlled with a greater flexibility.

### Second Embodiment

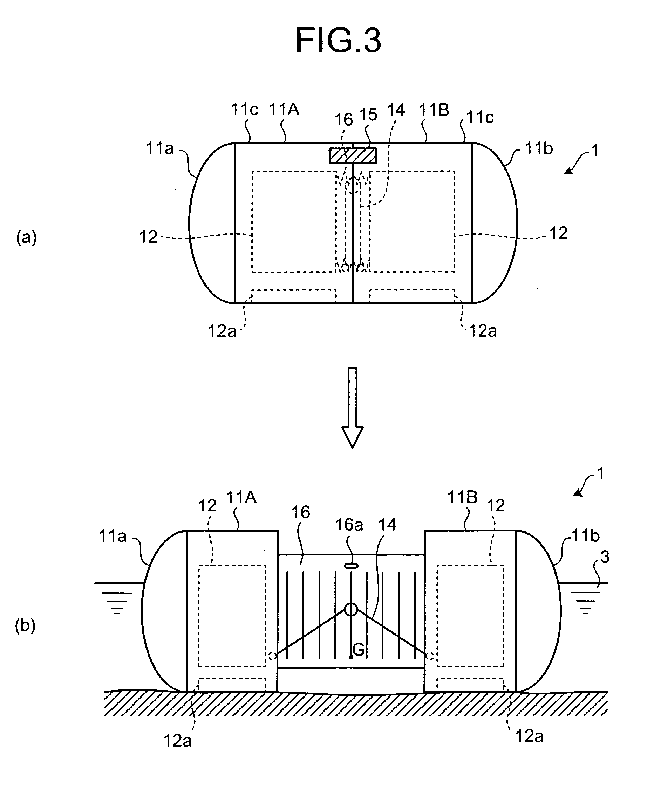

FIG. 3 is a schematic side view of examples of constitutions respectively before and after a volume increase of a capsule endoscope according to a second embodiment. The same part as shown in the first embodiment is shown by using the same reference symbol.

In the second embodiment, a volume changing unit 16 which is a tank formed of a hollow soft member of an accordion shape is provided instead of the volume changing unit 13 which is a tank having a rigid body of a cylindrical shape. The volume changing unit 16 is arranged between the casings 11A and 11B with respect to the capsule casing 11 and connected, to be stretchable along the longitudinal axis direction, to the bottomed parts of the casings 11A and 11B with an air-tight state maintained. By this, the volume changing unit 16 can be displaced to a storage position where the volume changing unit 16 shrinks to be stored between the casings 11A and 11B as shown at (a) in FIG. 3 and to an exposure position where the volume changing unit 16 stretches out to be exposed outward from the casings 11A and 11B as shown at (b) in FIG. 3 to increase a total casing volume of the capsule endoscope 1 and make the specific gravity less than one. To perform the displacement between the storage position and the exposure position, the volume changing unit 16 is provided, at a center part on a side surface, with one ventilation hole 16a through which an outer air is taken in and an inner air is discharged. Other constituents are the same as those in the first embodiment.

In this configuration, since the casings 11A and 11B are sealed by the sealing member 15, the volume changing unit 16 is maintained at the storage position against the memorized shape of the shape memory alloy spring 14 though the capsule endoscope 1 before starting an examination is under the condition of the normal temperature and the shape memory alloy spring 14 will try to present the memorized shape of the expanded open shape when the normal temperature is, for example, at about 25°C. By this, the capsule endoscope 1 can be maintained in a size of the state shown at (a) in FIG. 3 in which the casings 11A and 11B are coupled as one unit without causing the volume changing unit 16 to be exposed. The size is a normal capsule size and does not impair the swallowing performance from the oral cavity 2a.

At a time of starting the examination, the capsule endoscope 1 in the state as shown at (a) in FIG. 3 is swallowed from the oral cavity 2a and inserted into the stomach 2b. Upon the insertion into the subject body 2, the sealing member 15 dissolves by the stomach juices and the like and the sealed state is released. On this occasion, the capsule endoscope 1 is already inserted into the subject body 2 and subjected to a body temperature environment, the shape memory alloy spring 14 presents the memorized shape of the folded shape and the volume changing unit 16 is maintained at the shrinking storage position even when the sealed state is released. By this, the capsule endoscope 1 can be maintained in the size of the state shown at (a) in FIG. 3 in which the casings 11A and 11B are coupled as one unit without causing the volume changing unit 16 to be exposed. In this state, the specific gravity of the capsule endoscope 1 is not less than one.

Thereafter, when the time is right, the water 3 at 25°C is taken little by little to be fed into the stomach 2b. On this occasion, though the specific gravity of the capsule endoscope 1 is not less than one and the capsule endoscope 1 does not float on a surface of the water 3 fed into the stomach 2b, the capsule endoscope 1 becomes a state of lying on its side in which the ventilation hole 16a is oriented upward according to the position of the center of gravity G. Then, the shape memory alloy spring 14 of the lying capsule endoscope 1 comes to be present in the fed water 3 (the water 3 is assumed to be an amount not causing the capsule endoscope 1 to go under the water as shown at (b) in FIG. 3 at this point) and performs the displacement to present the memorized shape of the expanded open shape from the folded shape according to the temperature (25°C) of the water 3. In response to the displacement to the expanded open shape of the shape memory alloy spring 14, the casings 11A and 11B are displaced to a direction of becoming away from each other and the volume changing unit 16 connected between the casings 11A and 11B also makes the displacement to the exposure position as shown at (b) in FIG. 3 while expanding by absorbing an outer air from the ventilation hole 16a present at a position oriented to an upper direction and in the air in the stomach 2b. Due to the displacement of the volume changing unit 16 to the exposure position according to the stretching, the casing volume of the capsule endoscope 1 increases to be a state where the specific gravity is less than one.

When an appropriate amount of the water 3 is further fed into the stomach 2b under this condition, the capsule endoscope 1 whose specific gravity is lowered to be less than one can image and observe the wall of the stomach while floating on the surface of the fed water 3 as shown in FIG. 1.

After the examination is completed, the water 3 is discharged from the inside of the stomach 2b to a side of the small intestine. By this, the capsule endoscope 1 remaining in the stomach 2b is subjected to the environment of the body temperature of the subject body 2 and the shape memory alloy spring 14 makes the displacement from the expanded open shape to a direction to be folded and present the memorized shape of the bent shape. In response to this folding displacement of the shape memory alloy spring 14, the separated casings 11A and 11B are also displaced to a direction to be in contact with each other and the volume changing unit 16 connected between the casings 11A and 11B also makes the displacement to the storage position as shown at (a) in FIG. 3 while discharging the inner air from the ventilation hole 16a. By the displacement of the volume changing unit 16 to the storage position according to the shrinking, the size of the capsule endoscope 1 returns to a normal capsule size. Then, the capsule endoscope 1 moves to the side of the small intestine according to the subsequent peristalsis as usual and is finally excreted to an outside of the subject body 2. Hence, the case of the second embodiment shows the same advantageous effects as the case of the first embodiment.

Though the sealing by the sealing member 15 is adopted not to cause the capsule endoscope 1 before being swallowed to be displaced to a state of an increased volume in the first and the second embodiments, the capsule endoscope 1 may be, for example, stored under an environment of a constant temperature until a time right before being swallowed, so that the sealing member 15 may be eliminated. Or more specifically, the subject body 2 may hold the capsule endoscope 1 by hand and the like right before the swallowing to keep the capsule endoscope 1 under the body temperature environment, so that the sealing member 15 may be eliminated.

Besides, when there is a possibility that the water 3 comes into the inside of the volume changing units 13 and 16 respectively through the ventilation holes 13a and 16a due to a rotation and the like of the capsule endoscope 1, a sheet member such as a Gore-Tex (registered trademark) which allows only a gas to path through may be attached onto the ventilation holes 13a and 16a respectively of the volume changing units 13 and 16 to prevent an intrusion of the water 3. In addition, the displacement operation to the exposure position may be configured to be performed through an adjustment based on an inner pressure of the capsule endoscope 1 without providing the ventilation holes 13a and 16a. By this, the structure can be simplified.

Moreover, an application example is made to the compound-eye capsule endoscope, a single-eye capsule endoscope is similarly applicable. Specifically, in the case of using the single-eye capsule endoscope, the capsule casing may not be divided into two pieces like the casings 11A and 11B, a volume changing unit may be connected, to be displaced at an air-tight state, to one end which is not the side of capturing images, and a shape memory alloy spring connecting a capsule casing and a distal end side of the volume changing unit may be provided.

### INDUSTRIAL APPLICABILITY

As described, a capsule endoscope and an intra-stomach observing method according to the present invention are useful for a case of observing an inside of a subject body in a state of floating on a liquid fed into the subject body, and specifically suitable for observing a stomach wall with the capsule endoscope floating inside the stomach to which the liquid is fed.

## Claims

1. A capsule endoscope (1), comprising:
a capsule casing (11) which has therein contents (12) including an imaging unit and which is adapted to be inserted into an inside of a subject body (2),
wherein
the capsule casing (11) comprises a first casing (11A) and a second casing (11B); and
the capsule endoscope (1) further comprises:
a volume changing unit (13; 16) which is hollow and connected to the first and second casings (11A, 11B), and adapted to change a connection position of the first and second casings (11A, 11B) to change a volume of the capsule casing (11); and
an actuator (14) which is connected to the first and second casings (11A, 11B) and is adapted to change the connection position.

2. The capsule endoscope (1) according to claim 1, wherein the actuator (14) operates based on a change in a temperature of an outside of the capsule casing (11).

3. The capsule endoscope (1) according to claim 1, wherein the actuator (14) is a shape memory alloy spring.

4. The capsule endoscope (1) according to claim 1, wherein
the connection position is a storage position where the volume of the capsule casing (11) is minimum and an exposure position where the volume of the capsule casing (11) is not minimum,
a specific gravity of the capsule casing (11) with respect to a water (3) is not less than one at the storage position, and
a specific gravity of the capsule casing (11) with respect to the water (3) is less than one at the exposure position.

5. The capsule endoscope (1) according to claim 1, wherein the volume changing unit (13; 16) has a rigid body of a cylindrical shape and is connected to the capsule casing (11) to be freely movable inward and outward with respect to the first and second casings (11A, 11B).

6. The capsule endoscope (1) according to claim 1, wherein the volume changing unit (13; 16) is formed of a soft member of an accordion shape and connected to be freely stretchable inward and outward with respect to the first and second casings (11A, 11B).

7. The capsule endoscope (1) according to claim 1, wherein each of the two casings (11A, 11B) has a bottomed shape.

8. The capsule endoscope (1) according to claim 4, wherein the actuator (14) is formed by a shape memory alloy spring (14) which causes the volume changing unit (13; 16) to be displaced from the storage position to the exposure position at a temperature not more than a predetermined temperature.

9. The capsule endoscope (1) according to claim 8, further comprising a sealing member (15) which is formed of a material that dissolves when inserted into the subject body (2) and retains the volume changing unit (13; 16) at the storage position against a memorized shape, which causes the displacement to the exposure position, of the shape memory alloy spring (14).

10. The capsule endoscope (1) according to claim 1, wherein a ventilation hole (13a; 16a) is formed in the volume changing unit (13; 16).

11. The capsule endoscope (1) according to claim 10, wherein a center of gravity (G) of the capsule endoscope (1) is set at a position where the ventilation hole (13a; 16a) is oriented upward with respect to a gravity direction.

12. The capsule endoscope (1) according to claim 10, wherein a sheet member which allows only a gas to pass through is attached to the ventilation hole (13a; 16a).

## Patentansprüche

1. Kapselendoskop (1) aufweisend:
ein Kapselgehäuse (11), das darin Inhalte (12) aufweist, die eine Abbildungseinheit enthalten, und das dazu eingerichtet ist, in ein Inneres eines Subjektkörpers (2) eingeführt zu werden, wobei
das Kapselgehäuse (11) ein erstes Gehäuse (11A) und ein zweites Gehäuse (11B) aufweist; und
das Kapselendoskop (1) ferner aufweist:
eine Volumenänderungseinheit (13; 16), die hohl und mit den ersten und zweiten Gehäusen (11A, 11B) verbunden ist, und dazu eingerichtet ist, eine Verbindungsposition der ersten und zweiten Gehäuse (11A, 11B) zu ändern, um ein Volumen des Kapselgehäuses (11) zu ändern; und
einen Aktuator (14), der mit den ersten und zweiten Gehäusen (11A, 11B) verbunden und dazu eingerichtet ist, die Verbindungsposition zu ändern.

2. Kapselendoskop (1) nach Anspruch 1, bei dem der Aktuator (14) basierend auf einer Änderung der Temperatur außerhalb des Kapselgehäuses (11) arbeitet.

3. Kapselendoskop (1) nach Anspruch 1, bei dem der Aktuator (14) eine Formgedächtnislegierungsfeder ist.

4. Kapselendoskop (1) nach Anspruch 1, bei dem
die Verbindungsposition eine Speicherposition ist, bei der das Volumen des Kapselgehäuses (11) minimal ist und eine exponierte Position, bei der das Volumen des Kapselendoskops (11) nicht minimal ist,
eine spezifische Dichte des Kapselgehäuses (11) hinsichtlich eines Wassers (3) nicht kleiner als eine bei der Speicherposition ist und
eine spezifische Dichte des Kapselgehäuses (11) hinsichtlich des Wassers (3) kleiner als eine bei der exponierten Position ist.

5. Kapselendoskop (1) nach Anspruch 1, bei dem die Volumenänderungseinheit (13; 16) einen steifen Körper mit einer zylindrischen Form aufweist und mit dem Kapselgehäuse (11) verbunden ist, um hinsichtlich den ersten und zweiten Gehäusen (11A, 11B) einwärts und auswärts frei bewegbar zu sein.

6. Kapselendoskop (1) nach Anspruch 1, bei dem die Volumenänderungseinheit (13; 16) aus einem nachgiebigen Element mit einer Akkordeonform gebildet und verbunden ist, um hinsichtlich den ersten und zweiten Gehäusen (11A, 11B) einwärts und auswärts frei ausdehnbar zu sein.

7. Kapselendoskop (1) nach Anspruch 1, bei dem jedes der zwei Gehäuse (11A, 11B) eine Bodenform aufweist.

8. Kapselendoskop (1) nach Anspruch 4, bei dem der Aktuator (14) durch eine Formgedächtnislegierungsfeder (14) gebildet ist, die die Volumenänderungseinheit (13; 16) veranlasst, bei einer Temperatur, die eine vorbestimmte Temperatur nicht übersteigt, aus der Speicherposition in die exponierte Position versetzt zu werden.

9. Kapselendoskop (1) nach Anspruch 8, ferner aufweisend ein Abdichtungselement (15), das aus einem Material gebildet ist, das sich auflöst, wenn es in den Subjektkörper (2) eingeführt ist und die Volumenänderungseinheit (13; 16) in der Speicherposition gegen eine gespeicherte Form hält, was die Versetzung der Formgedächtnislegierungsfeder (14) in die exponierte Position verursacht.

10. Kapselendoskop (1) nach Anspruch 1, bei dem ein Belüftungsloch (13a; 16a) in der Volumenänderungseinheit (13; 16) gebildet ist.

11. Kapselendoskop (1) nach Anspruch 10, bei dem ein Schwerpunkt (G) des Kapselendoskops (1) in einer Position eingestellt ist, bei der sich das Belüftungsloch (13a; 16a) hinsichtlich einer Gravitationsrichtung nach oben orientiert.

12. Kapselendoskop (1) nach Anspruch 10, bei dem ein Folienelement, das nur Gas erlaubt, durch dieses zu strömen, an dem Belüftungsloch (13a; 16a) angebracht ist.

## Revendications

1. Endoscope (1) de type capsule, comprenant :
un boîtier (11) de capsule qui comporte dans celui-ci des contenus (12) incluant une unité d'imagerie et qui est adapté pour être inséré à l'intérieur d'un corps de sujet (2),
dans lequel
le boîtier (11) de capsule comprend un premier boîtier (11A) et un deuxième boîtier (11B) ; et
l'endoscope (1) de type capsule comprend en outre :
une unité (13 ; 16) de changement de volume qui est creuse et connectée aux premier et deuxième boîtiers (11A, 11B), et adaptée pour changer une position de connexion des premier et deuxième boîtiers (11A, 11B) pour changer un volume du boîtier (11) de capsule ; et
un actionneur (14) qui est connecté aux premier et deuxième boîtiers (11A, 11B) et est adapté pour changer la position de connexion.

2. Endoscope (1) de type capsule selon la revendication 1, dans lequel l'actionneur (14) fonctionne sur la base d'un changement de température d'un extérieur du boîtier (11) de capsule.

3. Endoscope (1) de type capsule selon la revendication 1, dans lequel l'actionneur (14) est un ressort en alliage à mémoire de forme.

4. Endoscope (1) de type capsule selon la revendication 1, dans lequel
la position de connexion est une position de stockage dans laquelle le volume du boîtier (11) de capsule est minimal et une position d'exposition dans laquelle le volume du boîtier (11) de capsule n'est pas minimal,
une gravité spécifique du boîtier (11) de capsule par rapport à de l'eau (3) n'est pas inférieure à celle au niveau de la position de stockage, et
une gravité spécifique du boîtier (11) de capsule par rapport à de l'eau (3) est inférieure à celle au niveau de la position d'exposition.

5. Endoscope (1) de capsule selon la revendication 1, dans lequel l'unité (13 ; 16) de changement de volume comporte un corps rigide d'une forme cylindrique et est connectée au boîtier (11) de capsule pour pouvoir se déplacer librement vers l'intérieur et vers l'extérieur par rapport aux premier et deuxième boîtiers (11A, 11B).

6. Endoscope (1) de type capsule selon la revendication 1, dans lequel l'unité (13 ; 16) de changement de volume est formée d'un élément mou en forme d'accordéon et connectée pour être librement extensible vers l'intérieur et vers l'extérieur par rapport aux premier et deuxième boîtiers (11A, 11B).

7. Endoscope (1) de type capsule selon la revendication 1, dans lequel chacun des deux boîtiers (11A, 11B) présente une forme arrondie.

8. Endoscope (1) de type capsule selon la revendication 4, dans lequel l'actionneur (14) est formé par un ressort (14) en alliage à mémoire de forme qui amène l'unité (13 ; 16) de changement de volume à être déplacée de la position de stockage à la position d'exposition à une température ne dépassant pas une température prédéterminée.

9. Endoscope (1) de type capsule selon la revendication 8, comprenant en outre un élément d'étanchéité (15) qui est formé d'un matériau qui se dissout lorsqu'inséré dans le corps (2) d'un sujet et retient l'unité (13 ; 16) de changement de volume à la position de stockage contre une forme mémorisée, qui provoque le déplacement vers la position d'exposition, du ressort (14) en alliage à mémoire de forme.

10. Endoscope (1) de type capsule selon la revendication 1, dans lequel un trou de ventilation (13a ; 16a) est formé dans l'unité (13 ; 16) de changement de volume.

11. Endoscope (1) de type capsule selon la revendication 10, dans lequel un centre de gravité (G) de l'endoscope (1) de type capsule est établi à une position à laquelle le trou de ventilation (13a ; 16a) est orienté vers le haut par rapport au sens de gravité.

12. Endoscope (1) de type capsule selon la revendication 10, dans lequel un élément de feuille qui permet uniquement à un gaz de traverser est fixé sur le trou de ventilation (13a ; 16a).
